(19) Europäisches Patentamt
European Patent Office
Office européen des brevets

(11) **EP 3 054 321 A1**

(12) **EUROPEAN PATENT APPLICATION**
published in accordance with Art. 153(4) EPC

(43) Date of publication:
**10.08.2016 Bulletin 2016/32**

(51) Int Cl.:
*G01T 1/24* (2006.01)  *G01T 1/161* (2006.01)
*H01L 31/08* (2006.01)

(21) Application number: **14851321.1**

(22) Date of filing: **30.09.2014**

(86) International application number:
**PCT/JP2014/076195**

(87) International publication number:
**WO 2015/050141 (09.04.2015 Gazette 2015/14)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **03.10.2013   JP 2013208107**

(71) Applicant: **Hitachi Aloka Medical, Ltd.**
**Mitaka-shi, Tokyo 181-8622 (JP)**

(72) Inventors:
• **KOMINAMI Shinya**
**Tokyo 100-8280 (JP)**
• **MOTAI Kumi**
**Tokyo 100-8280 (JP)**
• **KOBASHI Keiji**
**Tokyo 100-8280 (JP)**

(74) Representative: **MERH-IP Matias Erny Reichl
Hoffmann
Patentanwälte PartG mbB
Paul-Heyse-Strasse 29
80336 München (DE)**

(54) **SEMICONDUCTOR RADIATION DETECTOR, NUCLEAR MEDICINE DIAGNOSTIC DEVICE USING SAME, AND METHOD FOR PRODUCING SEMICONDUCTOR RADIATION DETECTOR**

(57)    A semiconductor radiation detector capable of measuring a $\gamma$-ray energy spectrum at 122 keV and 662 keV and having an energy resolution of no greater than 8 % with respect to 122 keV $\gamma$-rays, a nuclear medicine diagnostic device using the detector and a method for producing the semiconductor radiation detector are provided.

A semiconductor radiation detector 101 uses a semiconductor crystal 111 sandwiched by a cathode 112 and an anode 113. The semiconductor crystal 111 is configured from a thallium bromide single crystal in which the concentration of lead as an impurity is less than 0.1 ppm, the full width at half maximum of the (110) rocking curve in the X-ray diffraction in specimen tilting angle scan is no greater than 1.6 degrees, the full width at half maximum in specimen in-plane rotation angle scan is no greater than 3.5 degrees, and the full width at half maximum in X-ray incident angle scan is no greater than 1.3 degrees.

[Fig. 9]

| WAFER No. | Pb CONCENTRATION | HALF VALUE WIDTH (deg) OF α ROCKING CURVE | HALF VALUE WIDTH (deg) OF β ROCKING CURVE | HALF VALUE WIDTH (deg) OF θ ROCKING CURVE | RESOLUTION OF 122 keV (%) |
|---|---|---|---|---|---|
| 3 | <0.1 | 1.25 | 2.32 | 1.00 | 5 |
| 4 | <0.1 | 1.30 | 2.54 | 1.06 | 6.6 |
| 5 | <0.1 | 1.35 | 2.66 | 1.15 | 5.7 |
| 6 | <0.1 | 2.93 | 11.63 | 3.38 | 12 |
| 7 | <0.1 | 1.91 | 5.46 | 1.75 | 15 |
| 8 | <0.1 | 3.10 | 10.90 | 3.21 | 19 |
| 9 | <0.1 | 1.76 | 4.05 | 1.49 | 12 |

**Description**

Technical Field

The present invention relates to a semiconductor radiation detector, a nuclear medicine diagnostic device using the same, and a method for producing the semiconductor radiation detector.

Background Art

Recently, a nuclear medicine diagnostic device using a radiation detector for measuring radiation such as a $\gamma$-ray has been widely used. As typical nuclear medicine diagnostic devices, there are a gamma camera device, a Single Photon Emission Computed Tomography (SPECT) imaging device, a Positron Emission Tomography (PET) imaging device, and the like. In addition, there is an increased need, in terms of homeland security, for radiation detection devices for a dosimeter for anti-radiological bomb terrorism operations and the like using a radiation detection device.

Conventionally, these radiation detectors were formed by combining a scintillator and a photomultiplier, but, recently, a technique using a semiconductor radiation detector configured by semiconductor crystals of cadmium telluride, cadmium zinc telluride, gallium arsenide, thallium bromide, and the like has been attracting attention.

Since the semiconductor radiation detector is configured to convert a charge generated by interaction between the radiation and the semiconductor crystal into an electrical signal, there are various features in which conversion efficiency into the electrical signal is good, and reduction in size is possible compared to a case where the scintillator is used.

The semiconductor radiation detector includes a semiconductor crystal, a cathode that is formed one surface of the semiconductor crystal, and an anode that faces the cathode by sandwiching the semiconductor crystal. A charge, which is generated by applying a DC high voltage between the cathode and the anode when the radiation of an X-ray, a $\gamma$-ray, and the like is incident on the semiconductor crystal, is taken out by a signal from the cathode or the anode.

Here, in particular, in thallium bromide among the semiconductor crystals, a linear attenuation coefficient by a photoelectric effect is great compared to other semiconductor crystals of cadmium telluride, cadmium zinc telluride, gallium arsenide, and the like, and it is possible to obtain $\gamma$-ray sensitivity with a thin crystal equivalent to the other semiconductor crystals. Thus, the semiconductor radiation detector configured of thallium bromide and the nuclear medicine diagnostic device using the same can be further reduced in size compared to other semiconductor radiation detectors and nuclear medicine diagnostic devices using the same.

In addition, since thallium bromide is cheaper than the other semiconductor crystals of cadmium telluride, cadmium zinc telluride, gallium arsenide, and the like, the semiconductor radiation detector configured of thallium bromide and the nuclear medicine diagnostic device using the same can be cheaper than other semiconductor radiation detectors and the nuclear medicine diagnostic devices using the same.

In the semiconductor radiation detector formed by using thallium bromide as the semiconductor crystal, a $\gamma$-ray energy spectrum of 5.9 keV in which $^{55}$Fe is a radiation source and a $\gamma$-ray energy spectrum of 59.6 keV in which $^{241}$Am is a radiation source have been observed (for example, see Non-Patent Literature 1). However, in Non-Patent Literature 1, a $\gamma$-ray energy spectrum in which $^{57}$Co is a radiation source and a $\gamma$-ray energy spectrum in which $^{137}$Cs is a radiation source have not been observed.

In addition, in Fig. 1 of Non-Patent Literature 1, it is disclosed that a concentration of lead as an impurity contained in a thallium bromide crystal used in the radiation detector is $10^2$ ng/g (that is, 0.1 ppm).

In addition, in Table 1 of Non-Patent Literature 2, it is disclosed that a full width at half maximum in the X-ray incident angle scan of a rocking curve in the X-ray diffraction of the thallium bromide crystal is 0.094 deg to 0.58 deg.

Citation List

Non-Patent Literature

NPL 1: Nuclear Instruments and Methods in Physics Research Section-A, Vol. 591(2008), p. 209-212
NPL 2: Nuclear Instruments and Methods in Physics Research Section-A, Vol. 633(2011), p. 572-574

Summary of Invention

Technical Problem

However, as one of typical radioactive nuclides used in a radioactive pharmaceutical for nuclear medicine inspection by a gamma camera device, a SPECT imaging device and the like among the nuclear medicine diagnostic devices, there is $^{99m}$Tc. Main energy of the $\gamma$-ray emitted from $^{99m}$Tc is 141 keV and it is a prerequisite that the radiation detector used in the gamma camera device or the SPECT imaging device detects the $\gamma$-ray of 141 keV. Therefore, in order to examine performance of the radiation detector for the gamma camera device or the SPECT imaging device, $^{57}$Co mainly emitting the $\gamma$-ray of 122 keV of which energy is close to that of 141 keV is often used as a standard radiation source.

In addition, in the nuclear medicine inspection by the gamma camera or the SPECT imaging device, imaging of two nuclides may be necessary. For example, in a case where $^{99m}$Tc and $^{123}$I are used simultaneously as the radioactive pharmaceutical, if two nuclides can be imaged simultaneously, the efficiency of the nuclear medicine inspection is significantly improved. Since energy of the $\gamma$-ray emitted from $^{123}$I is 159 keV whereas main energy of the $\gamma$-ray emitted from $^{99m}$Tc is 141 keV, it is necessary to separate and detect a peak of 141 keV and a peak of 159 keV with the radiation detector in order to separate and detect the $\gamma$-ray emitted from $^{99m}$Tc and the $\gamma$-ray emitted from $^{123}$I with the radiation detector. As performance of the radiation detector therefor, energy resolution of equal to or less than 8% is required in 122 keV.

In addition, in the nuclear medicine inspection by the PET imaging device among the nuclear medicine diagnostic devices, it is a prerequisite to detect the $\gamma$-ray of 511 keV of a pair of energy emitted in the opposite direction of approximately 180 degrees when annihilating positron emitted from the radioactive pharmaceutical. Therefore, in order to examine the performance of the radiation detector for the PET imaging device, a $^{137}$Cs radiation source mainly emitting the $\gamma$-ray of 662 keV of which energy is close to that of 511 keV is often used as the standard radiation source.

However, in a case where the semiconductor radiation detector of thallium bromide is manufactured in the related art, both energy spectra of the $\gamma$-ray of 122 keV emitted from the $^{57}$Co radiation source and the $\gamma$-ray of 662 keV emitted from a $^{137}$Cs radiation source cannot be measured and it cannot be used as the radiation detector for the gamma camera device, the SPECT imaging device, and further the PET imaging device.

Lead of 0.1 ppm as an impurity is contained in the thallium bromide crystal used in the radiation detector described in Non-Patent Literature 1. Lead is the next element of thallium in the periodic table. Since lead and thallium are also metal elements, atomic radiuses thereof are defined by a metal bond radius and the atomic radius (metal bond radius) of lead is 0.175 nm whereas the atomic radius (metal bond radius) of thallium is 0.170 nm in accordance with a literature (Chemical Handbook Fundamentals, revised 5th edition, edited by the Chemical Society of Japan). Therefore, if lead atoms are entered as an impurity, substitutional solid solution is easily created by partially substituting thallium atoms and the lead atoms are likely to be valence II whereas the thallium atoms are likely to be valence I. Thus, a position in which the lead atoms are substituted is likely to be a defect as a crystal. In order to operate the thallium bromide crystal as the semiconductor radiation detector and to obtain high energy resolution, there is a need to collect a majority of the charge carrier that is made by causing the incident radiation to pass through, but it is considered that a trapping length, in which the charge carrier is trapped in defects in the crystal in which lead atoms can be substituted, is reduced and the $\gamma$-ray energy spectrum of 122 keV and 662 keV cannot be measured.

In the thallium bromide crystal described in Non-Patent Literature 2, the full width at half maximum in the X-ray diffraction in the X-ray incident angle scan of the rocking curve is 0.094 deg to 0.58 deg, but a full width at half maximum in specimen tilting angle scan, and a full width at half maximum in specimen in-plane rotation angle scan, are not measured. In addition, the concentration of an impurity, such as lead, is not described and $\gamma$-ray energy spectrum is also not described. Even if only the full width at half maximum in the X-ray diffraction in the X-ray incident angle scan of the rocking curve is controlled, the extent of the fluctuation of a crystal lattice of the thallium bromide single crystal is not exactly evaluated and if the fluctuation of the crystal lattice is great, a product of mobility and lifetime of a charge carrier in the crystal is reduced and $\gamma$-ray energy spectrum may not be observed. In addition, if the concentration of lead as an impurity is great, the charge carrier is trapped in the defect in the crystal in which the lead atoms are substituted, a trapping length is shortened, and $\gamma$-ray energy spectrum may not be also observed. That is, in addition to the full width at half maximum in the X-ray diffraction in the X-ray incident angle scan of the rocking curve, $\gamma$-ray energy spectrum may not be observed unless control is performed during crystal growth or a crystal is selected after crystal growth by evaluating the full width at half maximum in the specimen tilting angle scan, the full width at half maximum in the specimen in-plane rotation angle scan, and the concentration of lead as an impurity.

An object of the invention is to provide a semiconductor radiation detector, in which a $\gamma$-ray energy spectrum of 122 keV and 662 keV can be measured and energy resolution of equal to or less than 8% with respect to the $\gamma$-ray of 122 keV is obtained, a nuclear medicine diagnostic device using the same, and a method for producing the semiconductor radiation detector.

Solution to Problem

In order to solve the problem described above, the present invention provides a semiconductor radiation detector including a semiconductor crystal that is sandwiched by a cathode and an anode. The semiconductor crystal is configured from a thallium bromide single crystal in which a concentration of lead as an impurity is less than 0.1 ppm, a full width at half maximum of a (110) rocking curve in X-ray diffraction in specimen tilting angle scan is equal to or less than 1.6 deg, a full width at half maximum in specimen in-plane rotation angle scan is equal to or less than 3.5 deg, and a full width at half maximum in X-ray incident angle scan is equal to or less than 1.3 deg.

According to this configuration, since concentration of lead atoms in the thallium bromide single crystal is small, density of defects in the crystal in which lead atoms can be substituted to thallium atoms is reduced and a trapping length of a charge carrier can be increased. In addition, since fluctuation of the crystal lattice is small, it is possible to increase a product of mobility and lifetime of the charge carrier in the crystal. Therefore, as the radiation detector, it is possible to measure a $\gamma$-ray energy spectrum of 122 keV and 662 keV with high energy resolution.

Advantageous Effects of Invention

According to the invention, it is possible to measure the $\gamma$-ray energy spectrum of 122 keV and 662 keV and it is possible to obtain a semiconductor radiation detector, in which the energy resolution of equal to or less than 8% with respect to the $\gamma$-ray of 122 keV is obtained, and a nuclear medicine diagnostic device using the same.

Brief Description of Drawings

[Fig. 1] Fig. 1 is a perspective view illustrating a configuration of a semiconductor radiation detector according to an embodiment of the invention.
[Fig. 2] Fig. 2 is a sectional view illustrating the configuration of the semiconductor radiation detector according to an embodiment of the invention.
[Fig. 3] Fig. 3 is an explanatory view of lead concentration as an impurity of a semiconductor crystal used in the semiconductor radiation detector according to an embodiment of the invention and a semiconductor radiation detector of a comparison example.
[Fig. 4] Fig. 4 is a circuit diagram illustrating a circuit configuration in a case where radiation measurement is performed by using the semiconductor radiation detector according to an embodiment of the invention.
[Fig. 5] Fig. 5 is an explanatory view of a time change of a bias voltage applied to the semiconductor radiation detector according to an embodiment of the invention.
[Fig. 6] Fig. 6 is an explanatory view of a $\gamma$-ray energy spectrum measured by using the semiconductor radiation detector according to an embodiment of the invention.
[Fig. 7] Fig. 7 is an explanatory view of a $\gamma$-ray energy spectrum measured by using the semiconductor radiation detector of the comparison example.
[Fig. 8] Fig. 8 is an explanatory view of a specimen arrangement in an X-ray diffraction rocking curve of the semiconductor crystal used in the semiconductor radiation detector according to an embodiment of the invention.
[Fig. 9] Fig. 9 is an explanatory view illustrating a relationship between the lead concentration and a half value width of the X-ray diffraction rocking curve of the semiconductor crystals used in the detector of an embodiment of the invention and the detector of a comparison example, and energy resolution of the detector.
[Fig. 10] Fig. 10 is an explanatory view illustrating a relationship between a half value width of a specimen tilting angle ($\alpha$) rocking curve and the energy resolution of 122 keV of the semiconductor crystals used in the semiconductor radiation detector according to an embodiment of the invention and the semiconductor radiation detector of a comparison example.
[Fig. 11] Fig. 11 is an explanatory view illustrating a relationship between a half value width of a specimen in-plane rotation angle ($\beta$) rocking curve and the energy resolution of 122 keV of the semiconductor crystals used in the semiconductor radiation detector according to an embodiment of the invention and the semiconductor radiation detector of a comparison example.
[Fig. 12] Fig. 12 is an explanatory view illustrating a relationship between a half value width of an X-ray incident angle ($\theta$) rocking curve and the energy resolution of 122 keV of the semiconductor crystals used in the semiconductor radiation detector according to an embodiment of the invention and the semiconductor radiation detector of a comparison example.
[Fig. 13] Fig. 13 is an explanatory view of the $\gamma$-ray energy spectrum measured by using the semiconductor radiation detector according to an embodiment of the invention.

[Fig. 14] Fig. 14 is a configuration view of a nuclear medicine diagnostic device using the semiconductor radiation detector according to an embodiment of the invention.

[Fig. 15] Fig. 15 is a configuration view of the nuclear medicine diagnostic device using the semiconductor radiation detector according to an embodiment of the invention. Description of Embodiments

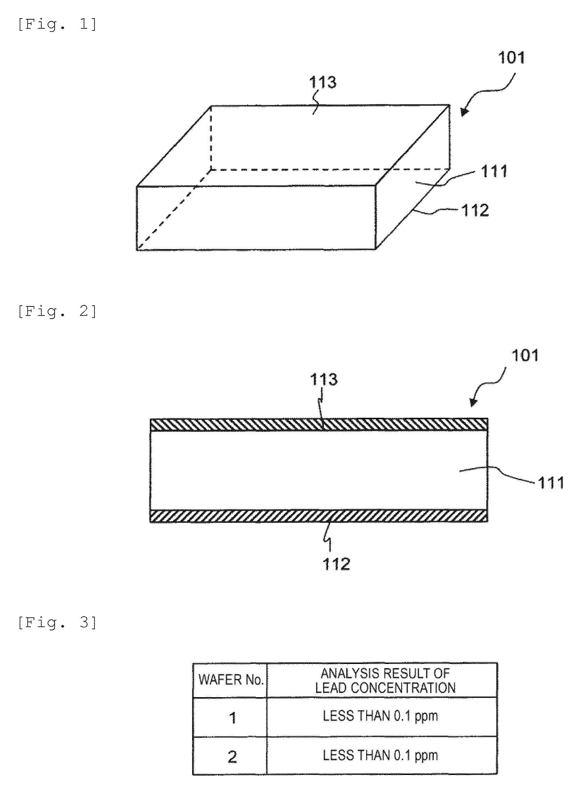

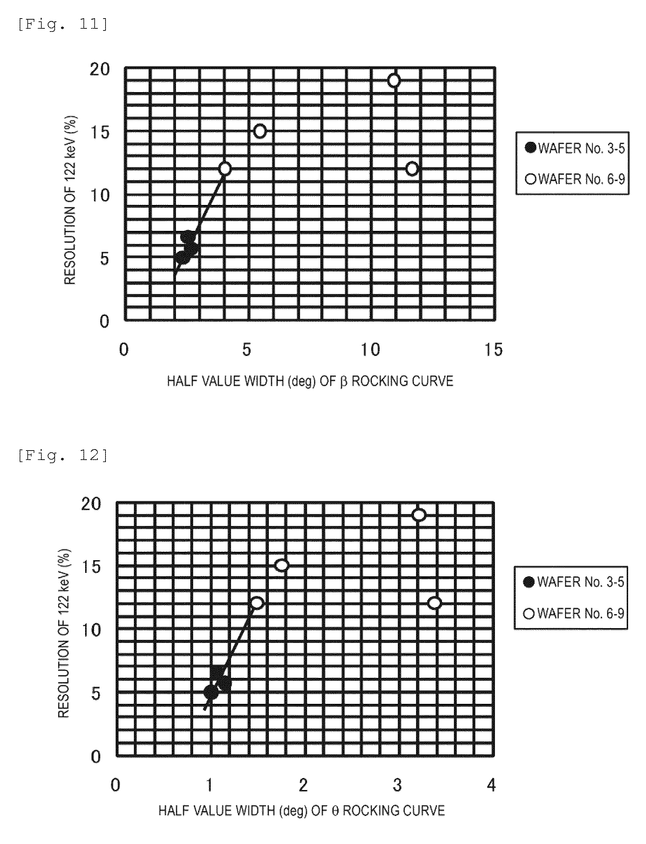

Hereinafter, a method for producing a semiconductor radiation detector, a configuration and an operation thereof, and a configuration and an operation of a nuclear medicine diagnostic device using the same according to one embodiment of the invention will be described with reference to Figs. 1 to 15.

Initially, the configuration of the semiconductor radiation detector according to the embodiment will be described with reference to Figs. 1 and 2.

Fig. 1 is a perspective view illustrating the configuration of the semiconductor radiation detector according to an embodiment of the invention and Fig. 2 is a sectional view.

A semiconductor radiation detector (hereinafter, simply referred to as "detector") 101 of the embodiment includes one semiconductor crystal 111 formed in a plate shape, a first electrode 112 disposed on one surface (lower surface) of the semiconductor crystal 111, and a second electrode 113 disposed on the other surface (upper surface).

The semiconductor crystal 111 forms a region in which a charge is generated by interacting with radiation ($\gamma$-ray and the like) and is formed by cutting a thallium bromide single crystal. The thallium bromide single crystal is grown by a single crystal growth apparatus after purification treatment of a raw material of thallium bromide of purity of 99.99% which is commercially available. Moreover, lead (Pb) as an impurity is contained in the raw material of thallium bromide of the purity of 99.99% which is commercially available. As a purification treatment method, there are a zone melting method, a vacuum evaporation method, and the like. In the embodiment, a process of purification is performed to aim to reduce concentration of lead as an impurity in a crystal. As a single crystal growth method, a vertical Bridgman method is used. A diameter of the crystal is approximately 3 inches. An extent of fluctuation of a crystal lattice of the grown crystal, a full width at half maximum of an X-ray diffraction rocking curve depends on a temperature gradient during growth, a growth rate, a crucible shape, thermal conductivity of a crucible, and the like. In the embodiment, the growing process of a single crystal ingot is performed to aim to suppress the fluctuation of the crystal lattice. In addition, in parallel therewith, a single crystal ingot of a comparison example is also grown by a growing process without paying attention to the extent of the fluctuation of the crystal lattice. As a result, two single crystal ingots of 3 inches of a single crystal ingot No. 1 of the embodiment and a single crystal ingot No. 2 of the comparison example are obtained. It is possible to obtain a thallium bromide single crystal wafer of 3 inches, of which a thickness is 0.5 mm, by polishing after cutting the single crystal ingot parallel to the crystal growth surface during growing the ingot by an annular saw slicing machine.

First, the concentration of lead as an impurity of the semiconductor crystal used in the semiconductor radiation detectors of the embodiment and the comparison example will be described with reference to Fig. 3.

Fig. 3 is an explanatory view of the lead concentration as an impurity of a semiconductor crystal used in the semiconductor radiation detector according to the embodiment of the invention and the semiconductor radiation detector of the comparison example.

In order to examine the concentration of lead as an impurity contained single crystal wafers No. 1 and No. 2 obtained from two types of the single crystal ingots No. 1 and No. 2 of 3 inches described above, a result obtained by performing Glow Discharge Mass Spectrometry (GDMS) is illustrated in Fig. 3. A detection limit of the concentration of lead is 0.1 ppm by the GDMS, lead is not detected in either of wafers No. 1 or No. 2, and the concentrations of lead are less than 0.1 ppm.

The semiconductor crystal 111 of a flat body of the embodiment illustrated in Figs. 1 and 2 and the semiconductor crystal of a flat body of the comparison example are obtained by dicing the single crystal wafer by, for example, dimensions of 5.5 mmx5.0 mm. Both concentrations of lead in the semiconductor crystal 111 manufactured from the wafer No. 1, and lead in the semiconductor crystal manufactured from the wafer No. 2, are less than 0.1 ppm. Thus, the concentration of lead is reduced compared to the thallium bromide crystal of the related art used in the radiation detector described in Non-Patent Literature 1. Therefore, a substitutional solid solution, in which lead atoms are substituted for a part of thallium atoms, is unlikely to be produced and density of defect in the crystal is also reduced. Thus, the charge carrier is unlikely to be trapped by the defect and it is possible to obtain a long trapping length.

The first electrode 112 and the second electrode 113 are formed by using one of gold, platinum, and palladium, and a thickness thereof is, for example, 50 nm. In addition, dimensions of the first electrode 112 and the second electrode 113 are, for example, 5.5 mmx5.0 mm.

Moreover, the dimensions of the semiconductor crystal 111, the first electrode 112, and the second electrode 113 described above are described as an example and are not limited to the dimensions described above respectively.

Next, manufacturing steps of the first electrode 112 and the second electrode 113 will be described.

First, gold, platinum, or palladium is deposited on one surface (lower surface and dimensions of 5.5 mm $\times$ 5.0 mm) of the semiconductor crystal 111 formed from thallium bromide of the flat body by 50 nm by an electron beam evaporating method and the first electrode 112 is formed.

Next, gold, platinum, or palladium is deposited on a surface (upper surface and dimensions of 5.5 mmx5.0 mm) opposite to the surface on which a first electrode of the semiconductor crystal 111 is by 50 nm by the electron beam evaporating method and the second electrode 113 is formed.

The detector 101 of the embodiment is obtained by using the semiconductor crystal 111 obtained by dicing the wafer No. 1 and the detector of the comparison example is obtained by using the semiconductor crystal obtained by dicing the wafer No. 2 through the steps described above.

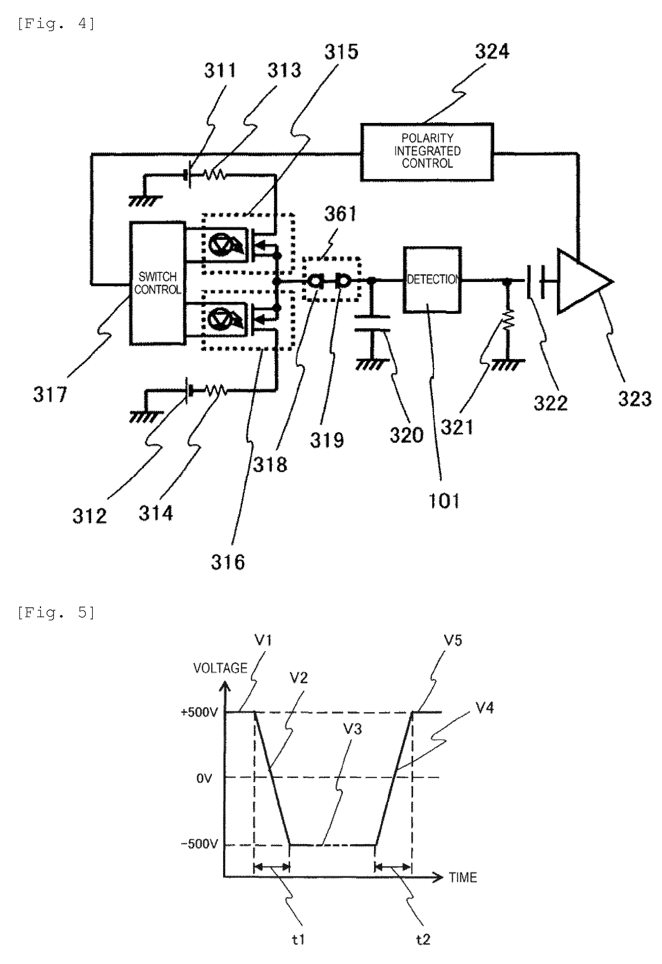

Next, in a case where radiation measurement is performed by using the semiconductor radiation detector according to the embodiment, a circuit configuration will be described with reference to Fig. 4.

Fig. 4 is a circuit diagram illustrating the circuit configuration in a case where the radiation measurement is performed by using the semiconductor radiation detector according to an embodiment of the invention.

In Fig. 4, a smoothing capacitor 320 that applies a voltage to the detector 101, a first DC power supply 311 that supplies a positive charge to one electrode of the smoothing capacitor 320, and a second DC power supply 312 that supplies a negative charge to the one electrode of the smoothing capacitor 320 are connected to the detector 101.

Furthermore, a first constant current diode 318 of which polarities of constant current characteristics are matched so as to allow a current to flow from the first DC power supply 311 to the one electrode of the smoothing capacitor 320 and a second constant current diode 319 of which polarities of constant current characteristics are matched so as to allow a current to flow from the one electrode of the smoothing capacitor 320 to the second DC power supply 312 are connected between both the first DC power supply 311 and the second DC power supply 312, and the detector 101.

Furthermore, a first photo MOS relay 315 is connected between the first DC power supply 311 and the one electrode of the smoothing capacitor 320, and a second photo MOS relay 316 is connected between the second DC power supply 312 and the one electrode of the smoothing capacitor 320.

Furthermore, a protection resistor 313 is connected between the first DC power supply 311 and the first photo MOS relay 315, and a protection resistor 314 is connected between the second DC power supply 312 and the second photo MOS relay 316. The protection resistors 313 and 314 are resistors for preventing overcurrent.

Opening and closing of the first photo MOS relay 315 and the second photo MOS relay 316 are controlled by a switch control device 317.

In addition, a bleeder resistor 321 and one electrode of a coupling capacitor 322 is connected to an output of the detector 101, and an amplifier 323 for amplifying a signal of the detector 101 is connected to the other electrode of the coupling capacitor 322. Furthermore, a polarity integrated control device 324 for controlling opening and closing of the photo MOS relays 315 and 316, and timing of polarity reversal of the amplifier 323 is connected to the switch control device 317 and the amplifier 323.

A negative electrode of the first DC power supply 311, a positive electrode of the second DC power supply 312, the other electrode other than the one electrode of the smoothing capacitor 320, and one electrode of the bleeder resistor 321 are respectively connected to a ground line.

Moreover, the first constant current diode 318 and the second constant current diode 319 are connected in series by reversing the polarities of the constant current characteristics with each other, and configure a constant current device 361. In this configuration, in a present general constant current diode used as the first constant current diode 318 and the second constant current diode 319, the constant current characteristics are created in a structure in which a source electrode and a gate electrode of a field effect transistor (FET) are short-circuited. Thus, p-n junction formed in the field effect transistor is biased in a forward direction and a large current flows when applying a reverse voltage. That is, current characteristics of the constant current diode have certain polarities. Thus, the first constant current diode 318 and the second constant current diode 319 are connected in series by reversing the polarities of the constant current characteristics with each other, thereby obtaining the constant current characteristics having no difference in polarities.

If the radiation of the $\gamma$-ray and the like is measured, a bias voltage for charge collection is applied between the first electrode 112 and the second electrode 113 of the detector 101 by the first DC power supply 311, or the second DC power supply 312 and the smoothing capacitor 320 (for example, +500 V or -500 V).

Here, since the semiconductor crystal 111 that is a member of the detector 101 is configured of thallium bromide, if the bias voltage of, for example, +500 V is continuously applied to the detector 101 by using the first DC power supply 311, deterioration of radiation measurement performance occurs and the energy resolution of the $\gamma$-ray is deteriorated in the semiconductor crystal 111 due to polarization, that is, deviation of the charge.

In order to prevent the polarization, it is necessary to periodically reverse the polarity of the bias voltage applied to the detector 101. That is, it is necessary to reverse the polarity, for example, from +500 V to -500 V and from -500 V to +500 V. The period of reversal is, for example, 5 minutes.

First, a case where the bias voltage of +500 V is applied to the detector 101 will be described. If the voltage of +500 V is directly applied from the first DC power supply 311 to the detector 101, noise occurs. Thus, the voltage is applied to the detector 101 using the smoothing capacitor 320.

The switch control device 317 closes the first photo MOS relay 315 and opens the second photo MOS relay 316 when applying a positive bias voltage to the detector 101.

The smoothing capacitor 320 is charged via the constant current device 361 and the voltage of the smoothing capacitor 320 is +500 V. Accordingly, the bias voltage applied to the detector 101 is also +500 V. Conversely, if the bias voltage of -500 V is applied to the detector 101, a negative DC bias voltage is supplied by the second DC power supply 312.

The switch control device 317 opens the first photo MOS relay 315 and closes the second photo MOS relay 316 when applying a negative bias voltage to the detector 101. The smoothing capacitor 320 is charged via the constant current device 361 and the voltage of the smoothing capacitor 320 is -500 V. The positive and negative bias voltages applied to the detector 101 are reversed by storing the positive charge or the negative charge in one electrode of the smoothing capacitor 320.

The polarity integrated control device 324 transmits command signals of "positive bias", "negative bias", "bias reversal from positive to negative", and "bias reversal from negative to positive" to the switch control device 317 and the amplifier 323 based on time information of polarity reversal of every 5 minutes. The switch control device 317 opens and closes the photo MOS relays 315 and 316 based on the command signals.

Here, a time change of the bias voltage applied to the semiconductor radiation detector according to the embodiment will be described with reference to Fig. 5.

Fig. 5 is an explanatory view of a time change of the bias voltage applied to the semiconductor radiation detector according to an embodiment of the invention.

In the embodiment, the bias voltage applied to the detector 101 is initially a voltage V1 (+500 V), is change to a voltage V3 (-500 V) due to periodical reversal of the bias voltage, and is returned to a voltage V5 (+500 V) again after 5 minutes.

When the bias voltage is reversed, the time change of voltages V2 and V4 in the middle thereof is a linear gradient. This is an effect of the constant current device 361. In addition, while reversing the bias voltage, an absolute value of the bias voltage is insufficient for collecting the charge and a $\gamma$-ray detection signal is not sufficiently removed. Interruption times (times t1 and t2 when the voltages V2 and V4 are applied) of the measurement are respectively 0.3 seconds. The interruption time of 0.3 seconds occurs during 5 minutes of measurement, but in a case where the semiconductor radiation detector is applied to the nuclear medicine diagnostic device or national security, it is not a problem because of a sufficiently short time.

If the $\gamma$-ray is incident on the detector 101 to which the bias voltage is applied, interaction occurs between the semiconductor crystal 111 configuring the detector 101 and the incident $\gamma$-ray and charges such as electrons and positive holes are generated.

The generated charge is output from the detector 101 as the $\gamma$-ray detection signal. The $\gamma$-ray detection signal is input into the amplifier 323 via the coupling capacitor 322. The bleeder resistor 321 prevents the charge from continuously storing in the coupling capacitor 322 and serves to make an output voltage of the detector 101 to be not too high. The amplifier 323 serves to convert the $\gamma$-ray detection signal that is a very small charge into a voltage and to amplify the voltage.

The $\gamma$-ray detection signal that is amplified by the amplifier 323 is converted into a digital signal by an analog-digital converter (not illustrated) of a later stage and is counted by a digital processing device (not illustrated) for each energy level of the $\gamma$-ray.

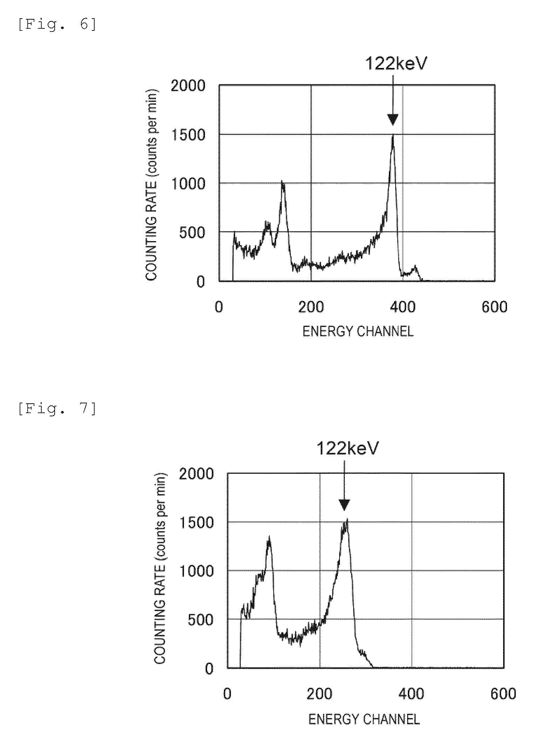

Next, the $\gamma$-ray energy spectrum of a $^{57}$Co radiation source measured by using the semiconductor radiation detectors of the embodiment and the comparison example will be described with reference to Figs. 6 and 7.

Figs. 6 and 7 are respectively an explanatory view of the $\gamma$-ray energy spectrum measured by using the semiconductor radiation detector according to an embodiment of the invention and an explanatory view of the $\gamma$-ray energy spectrum measured by using the semiconductor radiation detector of the comparison example.

Fig. 6 illustrates a measurement result of a case where the detector 101 is manufactured by using the semiconductor crystal 111 cut out from the wafer No. 1 described above. In addition, Fig. 7 illustrates a measurement result of a case where a detector is manufactured by using the semiconductor crystal cut out from the wafer No. 2 described above.

In Figs. 6 and 7, a horizontal axis indicates a channel number of an energy channel. The $\gamma$-rays having different energy levels are assigned to the energy channel of each number to correspond to each channel for each energy. For example, in Fig. 6, the $\gamma$-ray energy of substantially 122 keV is assigned to the energy channel in the vicinity of a substantially 380 channel. A vertical axis indicates a counting rate (counts per min) of the $\gamma$-ray of each energy channel.

In Fig. 6, a peak is observed in the counting rate of the energy channel corresponding to substantially 122 keV. The energy resolution in such a peak is expressed as follows.

```
      The energy resolution (%)=(channel number of a half value width of the peak)/(channel number just below the peak)×100

   ··· (Expression 1)
```

In the semiconductor radiation detector of the embodiment illustrated in Fig. 6, the energy resolution of 122 keV is substantially 5% and in the comparison example illustrated in Fig. 7, the energy resolution of 122 keV is substantially 15%.

As described above, it is possible to measure the $\gamma$-ray energy spectrum of 122 keV in both the detector 101 of the embodiment and the detector of the comparison example by making the concentration of lead as an impurity of the semiconductor crystal to be less than 0.1 ppm. However, there is a large difference in the energy resolution between the semiconductor radiation detector of the embodiment and the comparison example, and while it is possible to realize equal to or less than 8% in the detector 101 of the embodiment, a value worse than 8% is obtained in the detector of the comparison example. This is because the present application of the invention is made based on new knowledge of the present inventors that the energy resolution of the semiconductor radiation detector depends on the X-ray diffraction rocking curve of the semiconductor crystal.

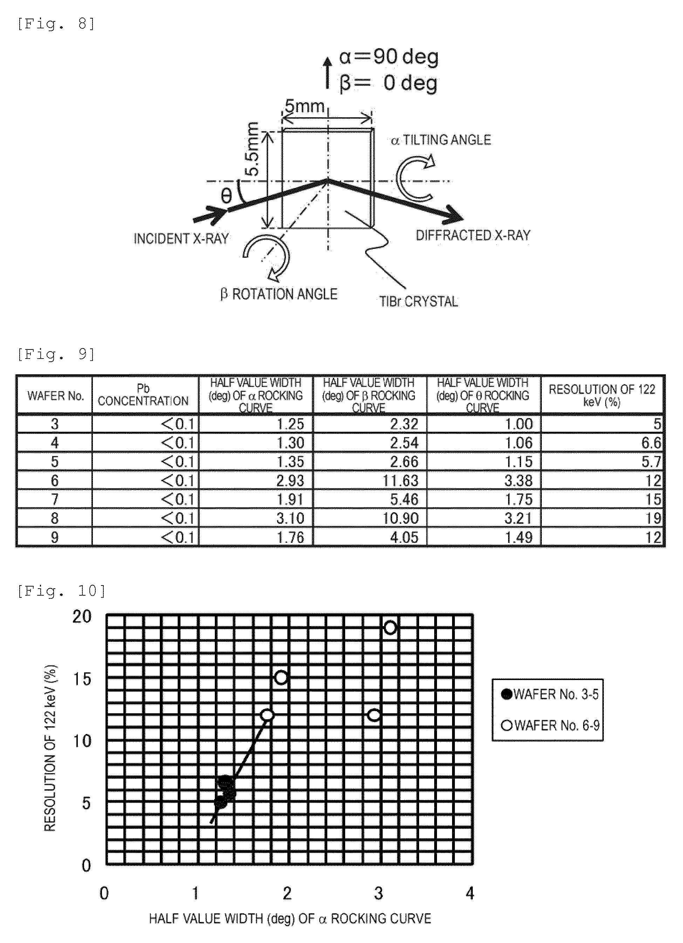

Here, a method for measuring the X-ray diffraction rocking curve of the semiconductor crystal 111 by using the semiconductor radiation detector 101 of the embodiment will be described with reference to Fig. 8. Fig. 8 is an explanatory view of a specimen arrangement in the X-ray diffraction rocking curve of the semiconductor crystal 111.

If the X-ray diffraction rocking curve is measured, the X-ray is incident on a specimen crystal (TIBr crystal) and a diffracted X-ray is detected. Three diffraction intensity profiles are obtained by fixing a detection angle of the diffracted X-ray and changing a tilting angle ($\alpha$) and a rotation angle ($\beta$) of the specimen, and an incident angle ($\theta$) of the incident X-ray respectively. In each of the three diffraction intensity profiles, a full width at half maximum of a diffraction intensity peak is a half value width of a $\alpha$ rocking curve, a half value width of a $\beta$ rocking curve, and a half value width of a $\theta$ rocking curve.

Next, the half value widths of the rocking curves of the semiconductor crystal 111 of the embodiment and the semiconductor crystal of the comparison example will be described with reference to Figs. 9 to 12. Fig. 9 is an explanatory view of the lead concentration, a half value width of the X-ray diffraction rocking curve, and the energy resolution of the semiconductor crystal 111 of the embodiment and the semiconductor crystal of the comparison example. In addition, Figs. 10 to 12 are respectively explanatory views illustrating a relationship between a half value width of the specimen tilting angle ($\alpha$) rocking curve, a half value width of a specimen in-plane rotation angle ($\beta$) rocking curve, a half value width of the X-ray incident angle ($\theta$) rocking curve of the semiconductor crystals used in the detector 101 according to the embodiment and in the detector of the comparison example, and the energy resolution of 122 keV of the detector 101 and the detector of the comparison example.

For a total of seven wafers, three wafers used as the semiconductor crystal 111 of the embodiment, that is, Nos. 3 to 5, and four wafers used as the semiconductor crystals of the comparison example, that is, Nos. 6 to 9, the lead concentration, the extent of the fluctuation of the crystal lattice, that is, the half value width of the X-ray diffraction rocking curve, and the energy resolution of 122 keV are evaluated. For a material of 7 wafers described above, a process of purification is performed to aim to reduce the concentration of lead as an impurity in the crystal and the concentrations of lead by GDMS of 7 wafers described above were all less than 0.1 ppm. Three measurement points, in which the half value width of the $\alpha$ rocking curve in the specimen tilting angle scan is equal to or less than 1.6 deg, the half value width of the $\beta$ rocking curve in the in-plane rotation angle scan is equal to or less than 3.5 deg, and the half value width of the $\theta$ the rocking curve in the X-ray incident angle scan is equal to or less than 1.3 deg, are the measurement results of the wafers No. 3 to No. 5 of the embodiment and the other four measurement points are the measurement results of the wafers No. 6 to No. 9 of the comparison example. As the semiconductor crystal in the embodiment, if the lead concentration is less than 0.1 ppm, the half value width of the $\alpha$ rocking curve is equal to or less than 1.6 deg, the half value width of the $\beta$ rocking curve is equal to or less than 3.5 deg, and the half value width of the $\theta$ rocking curve is equal to or less than 1.3 deg, it can be seen that the energy resolution of 122 keV of equal to or less than 8% is obtained.

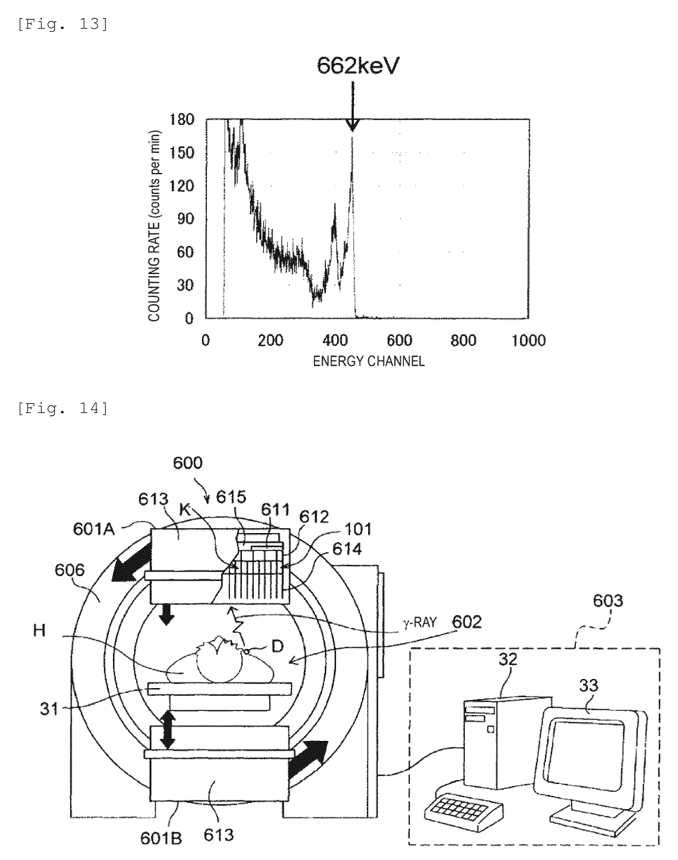

Next, the $\gamma$-ray energy spectrum of a $^{137}$Cs radiation source measured by using the semiconductor radiation detector 101 of the embodiment will be described using Fig. 13.

Fig. 13 illustrates a measurement result of a case where the detector 101 is manufactured by using the semiconductor crystal 111 cut out from the wafer No. 1 obtained from the ingot No. 1 described above.

In Fig. 13, a horizontal axis indicates the channel number of the energy channel. A vertical axis indicates a counting rate (counts per min) of the $\gamma$-ray of each energy channel.

In Fig. 13, the energy resolution of 662 keV is substantially 4%.

As described above, the detector 101 of the embodiment illustrated in Fig. 1 is configured by using the semiconductor crystal 111 cut out from wafer No. 1 and thereby energy spectrum of 662 keV is obtained with high energy resolution.

Therefore, in the detector 101 of the embodiment, radiation measurement performance of 122 keV and 662 keV is improved more than a case where the detector is configured by using the semiconductor crystal as the thallium bromide crystal of the related art described in Non-Patent Literature 1, the energy resolution of equal to or less than 8% is obtained in 122 keV, and high energy resolution is also obtained in 662 keV. This is because the semiconductor crystal 111 is configured of the thallium bromide single crystal in which the concentration of lead is less than 0.1 ppm, the full width at half maximum in the specimen tilting angle scan of the (110) rocking curve in the X-ray diffraction is equal to or less than 1.6 deg, the full width at half maximum in the specimen in-plane rotation angle scan is equal to or less than 3.5 deg, and the full width at half maximum in the X-ray incident angle scan is equal to or less than 1.3 deg in the detector 101 of the embodiment.

As the semiconductor crystal, the thallium bromide single crystal in which the concentration of lead is less than 0.1 ppm is used and thereby the concentration of the lead atoms in the thallium bromide single crystal is small. Thus, density of defects in the crystal in which lead atoms can be substituted to thallium atoms is reduced and a trapping length of a charge carrier can be increased. Therefore, as the radiation detector, it is possible to measure a $\gamma$-ray energy spectrum of 122 keV and 662 keV.

Here, as the semiconductor crystal, the thallium bromide single crystal, in which the concentration of lead is less than 0.1 ppm, is used and then it is also possible to use the thallium bromide single crystal in which the concentration of lead is equal to or less than the detection limit of lead in Glow Discharge Mass Spectrometry (GDMS). It is possible to measure the $\gamma$-ray energy spectrum of 122 keV and 662 keV as the radiation detector by using such a semiconductor crystal.

In addition, as the semiconductor crystal, the thallium bromide single crystal, in which the concentration of lead is less than 0.1 ppm, is used, and then as the semiconductor crystal, it is also possible to use the thallium bromide single crystal, in which the concentration of lead is 0.0 ppm. Here, the concentration of lead is 0.0 ppm, and then numbers equal to or less than two significant digit numbers may be any value, for example, the concentration of lead of 0.099 ppm, 0.09 ppm, 0.04 ppm, or equal to or less than 0.01 ppm are included. As the radiation detector, it is possible to measure the $\gamma$-ray energy spectrum of 122 keV and 662 keV by using such a semiconductor crystal.

Furthermore, as the semiconductor crystal, the thallium bromide single crystal, in which the concentration of lead is less than 0.1 ppm, is used and then as the semiconductor crystal, it is also possible to use the thallium bromide single crystal without the substitutional solid solution of lead. This is because if the concentration of lead is low to the point of being less than 0.1 ppm, the substitutional solid solution is not formed by substituting a part of the thallium atoms by lead as an impurity and defects do not occur. Thus, the charge carrier is unlikely to be trapped and the trapping length increases. Therefore, as the radiation detector, it is possible to measure the $\gamma$-ray energy spectrum of 122 keV and 662 keV by using such a semiconductor crystal.

Furthermore, as the semiconductor crystal, the thallium bromide single crystal, in which the concentration of lead is less than 0.1 ppm, is used and then as the semiconductor crystal, it is also possible to use the thallium bromide single crystal without defects in which the charge carrier is trapped. This is because if the concentration of lead is low less than 0.1 ppm, the substitutional solid solution is not formed by substituting a part of the thallium atoms by lead as an impurity and defects in which the charge carrier is trapped do not occur. Thus, the charge carrier is unlikely to be trapped and the trapping length increases. Therefore, as the radiation detector, it is possible to measure the $\gamma$-ray energy spectrum of 122 keV and 662 keV by using such a semiconductor crystal.

In addition, as the semiconductor crystal, the thallium bromide single crystal, in which the full width at half maximum in the specimen tilting angle scan is equal to or less than 1. 6 deg, the full width at half maximum in the specimen in-plane rotation angle scan is equal to or less than 3.5 deg, the full width at half maximum in the X-ray incident angle scan is equal to or less than 1.3 deg of the (110) rocking curve in the X-ray diffraction, is used. Thus, it is possible to reduce fluctuation of the crystal lattice in the single crystal and to increase a product of mobility and lifetime of the charge carrier. Therefore, as the radiation detector, it is possible to measure a $\gamma$-ray energy spectrum of 122 keV with high energy resolution, that is, the energy resolution of equal to or less than 8%.

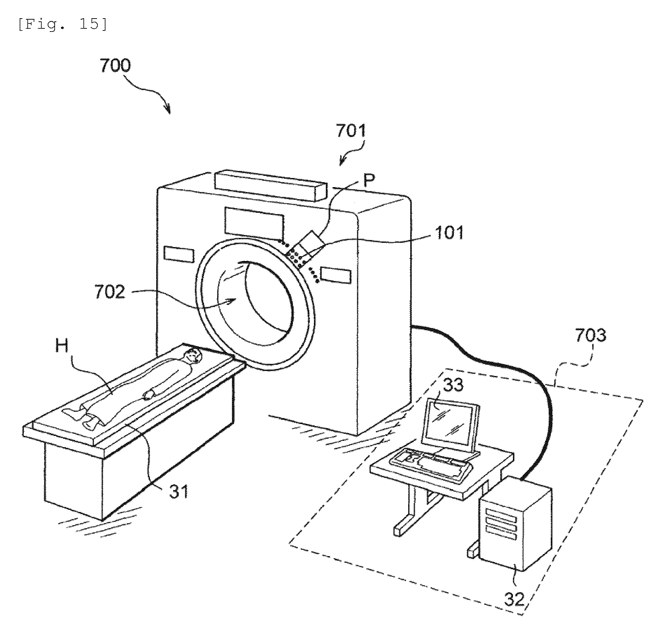

Next, a configuration of the nuclear medicine diagnostic device using the semiconductor radiation detector according to the embodiment will be described with reference to Figs. 14 and 15.

Figs. 14 and 15 are configuration views of the nuclear medicine diagnostic devices using the semiconductor radiation detectors according to an embodiment of the invention.

First, a case where the detector 101 of the embodiment is applied to a Single Photon Emission Computed Tomography imaging device (SPECT imaging device) 600 as the nuclear medicine diagnostic device will be described with reference to Fig. 14.

In Fig. 14, the SPECT imaging device 600 includes two radiation detection blocks 601A and 601B which are disposed above and below so as to surround a cylindrical measurement region 602 in a center portion, a rotation support base 606, a bed 31, and an image information creating device 603.

Here, the radiation detection block 601A disposed above includes a plurality of radiation measurement units 611, a unit support member 615, and a light-shielding and electromagnetic shield 613. The radiation measurement units 611 includes a plurality of semiconductor radiation detectors 101, a substrate 612, and a collimator 614. In addition, the radiation detection block 601B disposed below also has the same configuration. In addition, the image information creating device 603 is configured from a data processing device 32 and a display device 33.

The radiation detection blocks 601A and 601B are disposed at positions shifted by 180° in a circumferential direction in the rotation support base 606. Specifically, unit support members 615 (only one is illustrated) of the radiation detection blocks 601A and 601B are respectively attached to the rotation support base 606 at positions shifted by 180° in the circumferential direction. Then, the plurality of radiation measurement units 611 including the substrate 612 are attached to the unit support member 615 to be detachable.

The plurality of detectors 101 are respectively disposed in a region K partitioned by the collimator 614 in multiple stages in a state of being attached to the substrate 612. The collimator 614 forms a large number of radiation passages which are formed of a radiation shielding material (for example, lead, tungsten, and the like) and through which the radiation (for example, the $\gamma$-ray) passes.

The entirety of the substrate 612 and the collimator 614 are disposed within the light-shielding and electromagnetic shield 613 provided in the rotation support base 606. The light-shielding and electromagnetic shield 613 blocks the effect of electromagnetic waves other than the $\gamma$-ray to the detector 101 and the like.

In such a SPECT imaging device 600, the bed 31, on which a subject H to whom the radioactive pharmaceutical is administered is mounted, is moved and the subject H is moved between a pair of the radiation detection blocks 601A and 601B. Then, the rotation support base 606 is rotated and thereby each of the radiation detection blocks 601A and 601B turns around the subject H, and detection is started.

Then, if the $\gamma$-ray is emitted from an accumulation part (for example, affected part) D within the subject H in which the radioactive pharmaceutical is accumulated, the emitted $\gamma$-ray is incident on the corresponding detector 101 through the radiation passage of the collimator 614. Then, the detector 101 outputs a $\gamma$-ray detection signal. The $\gamma$-ray detection signal is counted by the data processing device 32 for each $\gamma$-ray energy level and information thereof and the like are displayed on the display device 33.

Moreover, in Fig. 14, the radiation detection blocks 601A and 601B rotate as indicated by bold arrows while being supported by the rotation support base 606 and perform imaging and measurement while changing an angle from the subject H. In addition, the radiation detection blocks 601A and 601B are able to move up and down as indicated by thin arrows, and can change the distance from the subject H.

The detector 101 used in such a SPECT imaging device 600 can measure the $\gamma$-ray energy spectrum of 122 keV with high energy resolution of equal to or less than 8% while using thallium bromide as the semiconductor crystal. Therefore, it is possible to provide the SPECT imaging device capable of imaging simultaneously two nuclides of $^{99m}$Tc emitting the $\gamma$-ray of 141 keV and $^{123}$I emitting the $\gamma$-ray of 159 keV that are small in size, inexpensive, and typical radioactive nuclides used in the radioactive pharmaceutical for the nuclear medicine inspection with high energy resolution.

Next, a case where the detector 101 of the embodiment is applied to a PET imaging device 700 as the nuclear medicine diagnostic device will be described with reference to Fig. 15.

The detector 101 of the embodiment is not limited to the SPECT imaging device 600 and can be used in a gamma camera device, the PET imaging device, and the like as the nuclear medicine diagnostic device.

In Fig. 15, the Positron Emission Tomography imaging device (PET imaging device) 700 includes an imaging device 701 which has a cylindrical measurement region 702 in a center portion, the bed 31 which supports a subject H and is capable of moving in a longitudinal direction, and an image information creating device 703. Moreover, the image information creating device 703 is configured to include the data processing device 32 and the display device 33.

A substrate P on which a large number of the detectors 101 are mounted is disposed in the imaging device 701 so as to surround the measurement region 702.

In the PET imaging device 700 described above, an application specific integrated circuit for a digital circuit (ASIC for a digital circuit (not illustrated)) having a data processing function and the like are provided, a packet having an energy value of the $\gamma$-ray, time, and a detection channel identification (ID) of the detector 101 is created, and the created packet is input into the data processing device 32.

The $\gamma$-ray emitted from the body of the subject H due to the radioactive pharmaceutical is detected by the detector 101 during inspection. That is, a pair of the $\gamma$-rays are emitted in the opposite direction of substantially 180 degrees and the $\gamma$-rays are detected by a separate detection channel of a large numbers of detectors 101 when positron emitted from the radioactive pharmaceutical for PET imaging is annihilated. The detected $\gamma$-ray detection signal is input into the digital ASIC, signal processing is performed as described above, and position information of the detection channel detecting the $\gamma$-ray and detection time information of the $\gamma$-ray are input into the data processing device 32.

Then, a pair of the $\gamma$-rays generated by annihilation of one positron is counted (counted simultaneously) as one by the data processing device 32 and positions of two detection channels detecting a pair of the $\gamma$-rays are specified

based on the position information. In addition, the data processing device 32 creates tomographic image information (image information) of the subject H in an accumulation position of the radioactive pharmaceutical, that is, a tumor position by using the count value obtained by counting simultaneously and the position information of the detection channel. The tomographic image information is displayed on the display device 33.

The detector 101 used in the PET imaging device 700 described above can measure the γ-ray energy spectrum of 662 keV with high energy resolution while using thallium bromide as the semiconductor crystal. Therefore, it is possible to provide the PET imaging device capable of detecting the γ-ray of 511 keV that are small in size, inexpensive, and emitted by positrons generated from the radioactive pharmaceutical for the PET inspection with high energy resolution.

As described above, according to the embodiment, it is possible to measure the γ-ray energy spectrum of 122 keV and 662 keV with high energy resolution by the radiation detector while using thallium bromide as the semiconductor crystal configuring the radiation detector. Thus, it is possible to provide the semiconductor radiation detector that is small in size and inexpensive, and has high energy resolution and the nuclear medicine diagnostic device on which the semiconductor radiation detector is mounted.

Moreover, the semiconductor radiation detector of the invention and the nuclear medicine diagnostic device on which the semiconductor radiation detector is mounted can image the radioactive pharmaceutical with high energy resolution, and can be reduced in size and inexpensive. Thus, it contributes to propagation of the devices and the devices are widely used and employed in this field.

Reference Signs List

31 bed
32 data processing device
33 display device
101 semiconductor radiation detector (detector)
111 semiconductor crystal
112 first electrode
113 second electrode
311 first DC power supply
312 second DC power supply
313, 314 protection resistor
315 first photo MOS relay
316 second photo MOS relay
317 switch control device
318 first constant current diode
319 second constant current diode
320 smoothing capacitor
321 bleeder resistor
322 coupling capacitor
323 amplifier
324 polarity integrated control device
361 constant current device
600 SPECT imaging device
601A, 601B radiation detection block
602, 702 measurement region
603, 703 image information creating device
606 rotation support base
611 radiation measurement unit
612 substrate
613 light-shielding and electromagnetic shield
614 collimator
615 unit support member
700 PET imaging device
701 imaging device
D accumulation part
H subject
K region partitioned by collimator

P substrate

**Claims**

1. A semiconductor radiation detector comprising:

   a semiconductor crystal that is sandwiched by a cathode and an anode,
   wherein the semiconductor crystal is configured from a thallium bromide single crystal in which a concentration of lead as an impurity is less than 0.1 ppm, a full width at half maximum of a (110) rocking curve in X-ray diffraction in specimen tilting angle scan is equal to or less than 1.6 deg, a full width at half maximum in specimen in-plane rotation angle scan is equal to or less than 3.5 deg, and a full width at half maximum in X-ray incident angle scan is equal to or less than 1.3 deg.

2. A semiconductor radiation detector comprising:

   a semiconductor crystal that is sandwiched by a cathode and an anode,
   wherein the semiconductor crystal is configured from a thallium bromide single crystal in which a concentration of lead as an impurity is equal to or less than a detection limit of a lead concentration by a glow discharge mass spectrometry (GDMS), a full width at half maximum of a (110) rocking curve in X-ray diffraction in specimen tilting angle scan is equal to or less than 1.6 deg, a full width at half maximum in specimen in-plane rotation angle scan is equal to or less than 3.5 deg, and a full width at half maximum in X-ray incident angle scan is equal to or less than 1.3 deg.

3. A semiconductor radiation detector comprising:

   a semiconductor crystal that is sandwiched by a cathode and an anode,
   wherein the semiconductor crystal is configured from a thallium bromide single crystal in which a concentration of lead as an impurity is 0.0 ppm, a full width at half maximum of a (110) rocking curve in X-ray diffraction in specimen tilting angle scan is equal to or less than 1.6 deg, a full width at half maximum in specimen in-plane rotation angle scan is equal to or less than 3.5 deg, and a full width at half maximum in X-ray incident angle scan is equal to or less than 1.3 deg.

4. The semiconductor radiation detector according to any one of claims 1 to 3,
   wherein the cathode and the anode are configured of at least one or more metals of gold, platinum, and palladium.

5. A method for producing a semiconductor detector including a semiconductor crystal that is sandwiched by a cathode and an anode, the method comprising:

   selecting a thallium bromide single crystal as the semiconductor crystal in which a concentration of lead as an impurity is less than 0.1 ppm, a full width at half maximum of a (110) rocking curve in X-ray diffraction in specimen tilting angle scan is equal to or less than 1.6 deg, a full width at half maximum in specimen in-plane rotation angle scan is equal to or less than 3.5 deg, and a full width at half maximum in X-ray incident angle scan is equal to or less than 1.3 deg.

6. A method for producing a semiconductor detector including a semiconductor crystal that is sandwiched by a cathode and an anode, the method comprising:

   selecting a thallium bromide single crystal as the semiconductor crystal in which a concentration of lead as an impurity is equal to or less than a detection limit of a lead concentration by a glow discharge mass spectrometry (GDMS), a full width at half maximum of a (110) rocking curve in X-ray diffraction in specimen tilting angle scan is equal to or less than 1.6 deg, a full width at half maximum in specimen in-plane rotation angle scan is equal to or less than 3.5 deg, and a full width at half maximum in X-ray incident angle scan is equal to or less than 1.3 deg.

7. A method for producing a semiconductor detector including a semiconductor crystal that is sandwiched by a cathode and an anode, the method comprising:

   selecting a thallium bromide single crystal as the semiconductor crystal in which a concentration of lead as an impurity is 0.0 ppm, a full width at half maximum of a (110) rocking curve in X-ray diffraction in specimen tilting angle scan is equal to or less than 1.6 deg, a full width at half maximum in specimen in-plane rotation angle scan is equal to or less than 3.5 deg, and a full width at half maximum in X-ray incident angle scan is equal to or less than 1.3 deg.

8. The method for producing a semiconductor radiation detector according to any one of claims 5 to 7, wherein in a case where the semiconductor crystal is manufactured, a surface for depositing the cathode and the anode is formed by being cut parallel to a crystal growth surface when growing an ingot as a raw material of the semiconductor crystal.

9. A nuclear medicine diagnostic device comprising:

a substrate on which a plurality of the semiconductor radiation detectors are mounted, which surrounds a measurement region into which a head supporting a subject is inserted, and which is disposed around the measurement region; and
an image information creating device that generates an image by using information obtained based on a radiation detection signal output from the plurality of semiconductor radiation detectors of the substrate,
wherein the semiconductor radiation detector is the semiconductor radiation detector according to any one of claims 1 to 3.

[Fig. 1]

[Fig. 2]

[Fig. 3]

| WAFER No. | ANALYSIS RESULT OF LEAD CONCENTRATION |
|-----------|----------------------------------------|
| 1 | LESS THAN 0.1 ppm |
| 2 | LESS THAN 0.1 ppm |

[Fig. 4]

311  313  315          324

POLARITY
INTEGRATED
CONTROL

361

SWITCH
CONTROL

DETECTION

317

312  314

316          318  319  320  321  322  323

101

[Fig. 5]

V1          V5

VOLTAGE   V2          V4
+500V          V3

0V

-500V

TIME

t1          t2

[Fig. 6]

[Fig. 7]

[Fig. 8]

[Fig. 9]

| WAFER No. | Pb CONCENTRATION | HALF VALUE WIDTH (deg) OF α ROCKING CURVE | HALF VALUE WIDTH (deg) OF β ROCKING CURVE | HALF VALUE WIDTH (deg) OF θ ROCKING CURVE | RESOLUTION OF 122 keV (%) |
|---|---|---|---|---|---|
| 3 | <0.1 | 1.25 | 2.32 | 1.00 | 5 |
| 4 | <0.1 | 1.30 | 2.54 | 1.06 | 6.6 |
| 5 | <0.1 | 1.35 | 2.66 | 1.15 | 5.7 |
| 6 | <0.1 | 2.93 | 11.63 | 3.38 | 12 |
| 7 | <0.1 | 1.91 | 5.46 | 1.75 | 15 |
| 8 | <0.1 | 3.10 | 10.90 | 3.21 | 19 |
| 9 | <0.1 | 1.76 | 4.05 | 1.49 | 12 |

[Fig. 10]

[Fig. 11]

[Fig. 12]

[Fig. 13]

[Fig. 14]

[Fig. 15]

## INTERNATIONAL SEARCH REPORT

International application No.

PCT/JP2014/076195

### A. CLASSIFICATION OF SUBJECT MATTER
*G01T1/24*(2006.01)i, *G01T1/161*(2006.01)i, *H01L31/08*(2006.01)i

According to International Patent Classification (IPC) or to both national classification and IPC

### B. FIELDS SEARCHED

Minimum documentation searched (classification system followed by classification symbols)
G01T1/24, G01T1/161, H01L31/08

Documentation searched other than minimum documentation to the extent that such documents are included in the fields searched
| | | | |
|---|---|---|---|
| Jitsuyo Shinan Koho | 1922–1996 | Jitsuyo Shinan Toroku Koho | 1996–2014 |
| Kokai Jitsuyo Shinan Koho | 1971–2014 | Toroku Jitsuyo Shinan Koho | 1994–2014 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | JP 2005-223009 A (Hitachi, Ltd.), 18 August 2005 (18.08.2005), entire text; all drawings & US 2006/0065847 A1 & EP 1584948 A2 | 1-9 |
| A | JP 2013-156048 A (Hitachi, Ltd.), 15 August 2013 (15.08.2013), entire text; all drawings & WO 2013/111844 A1 | 1-9 |
| A | JP 2001-349948 A (NEC Corp.), 21 December 2001 (21.12.2001), paragraph [0012] (Family: none) | 1-9 |

☒ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 23 October, 2014 (23.10.14) | 04 November, 2014 (04.11.14) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
|---|
| PCT/JP2014/076195 |

C (Continuation).   DOCUMENTS CONSIDERED TO BE RELEVANT

| Category* | Citation of document, with indication, where appropriate, of the relevant passages | Relevant to claim No. |
|---|---|---|
| A | JP 2009-286856 A (Kabushiki Kaisha Fukuda Crystal Laboratory), 10 December 2009 (10.12.2009), paragraph [0020] (Family: none) | 1-9 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- *Nuclear Instruments and Methods in Physics Research Section-A,* 2008, vol. 591, 209-212
- *Nuclear Instruments and Methods in Physics Research Section-A,* 2011, vol. 633, 572-574

- Chemical Handbook Fundamentals. Chemical Society of Japan